# EUROPÄISCHE PATENTSCHRIFT

(11) **EP 0 668 741 B1**
(45) Veröffentlichungstag und Bekanntmachung des Hinweises auf die Patenterteilung: **29.01.1997**
(21) Anmeldenummer: 93924510.6
(22) Anmeldetag: 10.11.1993
(51) Int. Cl.: A61B 6/14, A61B 6/00, H05G 1/26

(54) **VERFAHREN UND VORRICHTUNG ZUR ABNAHME- UND KONSTANZPRÜFUNG FILMLOSER DENTAL-RÖNTGENGERÄTE**
PROCESS AND DEVICE FOR ACCEPTANCE AND REGULAR TESTING OF FILMLESS DENTAL RADIOGRAPHIC EQUIPMENT
PROCEDE ET DISPOSITIF PERMETTANT DE PRATIQUER DES ESSAIS DE RECEPTION ET DE CONSTANCE D'APPAREILS RADIOGRAPHIQUES DENTAIRES SANS FILM

(30) Priorität: 12.11.1992 DE 4238268
(43) Veröffentlichungstag der Anmeldung: 30.08.1995
(73) Patentinhaber: SIEMENS AKTIENGESELLSCHAFT, 80333 München (DE)
(72) Erfinder: SCHULZE-GANZLIN, Ulrich, D-64653 Lorsch (DE); BLASCHKA, Eriks, D-69469 Weinheim (DE); PLÖTZ, Josef, D-64625 Bensheim (DE)
(86) Internationale Anmeldenummer: DE9301073
(87) Internationale Veröffentlichungsnummer: WO9410909

(56) Entgegenhaltungen:
- EP-A- 0 460 749
- EP-A- 0 506 559
- US-A- 4 352 020

## Beschreibung

Zur Konstanzprüfung von Dental-Röntgengeräten, die in Verbindung mit einem Röntgenfilm betrieben werden, ist aus DE-36 42 565 eine Vorrichtung bekannt, die einen Prüfkörper mit einer strahlenabsorbierenden Treppe aus mehreren, unterschiedlich stark absorbierenden Elementen umfaßt. Im Prüfkörper befindet sich eine Vertiefung, in die ein Röntgenfilm einlegbar ist. Die Prüfvorrichtung kann auf den Tubus eines Röntgengerätes aufgesetzt werden und hält zu diesem Zweck eine geeignete Zentrierhilfe.

Aus US-PS 4 352 020 ist weiterhin ein Computertomograph bekannt, bei dem zur Konstanzprüfung der charakteristischen Eigenschaften der Detektoren in den Strahlengang des Röntgenstrahlers eine Vielzahl von sogenannten Phantomelementen mit unterschiedlicher Absorption gebracht werden können. Um Abweichungen der Detektoren festzustellen, werden diese einmal ohne und danach mit in den Strahlengang eingebrachter Phantomelemente mit Strahlung beaufschlagt. Die so erhaltenen Vergleichswerte werden in einer Recheneinheit ausgewertet und auf einem Display angezeigt.

Bei filmlosen Dental-Röntgengeräten, bei denen das Röntgenbild von einem Sensor, z.B. einem CCD-Sensor, aufgenommen wird, besteht, wie bei der heutigen Röntgenfilmtechnik, die Notwendigkeit, den Sensor, und zwar einmal bei der Abnahme des Gerätes und später in regelmäßigen Abständen, einer Überprüfung zu unterziehen. Bei der in regelmäßigen Abständen vorzusehenden (Konstanz-)Prüfung, die üblicherweise vom Betreiber des Röntgengerätes durchgeführt wird, kann darauf geschlossen werden, ob das bilderzeugende System innerhalb festgelegter Grenzabweichungen konstant geblieben ist.

Der im Anspruch 1 angegebenen Erfindung liegt die Aufgabe zugrunde, ein für solche filmlose Dental-Röntgengeräte abgestimmtes Prüfverfahren anzugeben.

Ein die Erfindung tragendes Merkmal ist in der rechnergestützten Analyse der Meßergebnisse zu sehen. Dadurch kann eine subjektive, visuelle Beurteilung weiter unterstützt bzw. ergänzt werden.

Das erfindungsgemäß vorgeschlagene Verfahren ist sowohl für Dental-Röntgengeräte mit intraoral plazierbarem Sensor als auch für Dental-Röntgengeräte, mit denen Panoramaschichtaufnahmen bzw. Fernröntgenaufnahmen erstellt werden können, anwendbar. Demgemäß kann der Prüfmeßkörper in vorteilhafter Weise so ausgestaltet sein, daß er sowohl zur reproduzierbaren Halterung eines intraoral applizierbaren Sensors als auch zur Halterung an der Sekundärblende eines Panoramaschichtaufnahmegerätes geeignet ist.

In Anwendung bei Geräten mit intraoral applizierbarem Sensor ist es vorteilhaft, den Prüfmeßkörper mit einer Sensorhalterung auszustatten, die es erlaubt, den Sensor in einem engen definierten Abstand, beispielsweise von einigen wenigen Millimetern, vom Tubusrand zu haltern und in vorteilhafter Weise in bestimmten Winkelgrad-Schritten um die Tubusachse zu drehen. Die Sensorhalterung fixiert den Sensor in dem vorgegebenen Abstand zum Drehmittelpunkt.

Weitere vorteilhafte Ausgestaltungen und Weiterbildungen der Erfindung ergeben sich aus den Unteransprüchen und der nachfolgenden Beschreibung von Ausführungsbeispielen.

Es zeigen:
Figur 1 ein Blockschaltbild zur Erläuterung des Verfahrens,
Figur 2 die erfindungsgemäße Vorrichtung in Anwendung bei einem Röntgengerät mit intraoral applizierbarem Sensor, im Schnitt entlang der Linie II/II in Figur 3,
Figur 3 die Vorrichtung nach Figur 2 in Ansicht,
Figur 4 die Vorrichtung nach Figur 3 in einer um 45° gedrehten Stellung,
Figuren 5 und 6 die erfindungsgemäße Vorrichtung in Anwendung bei einem Panorama-Röntgengerät,
Figuren 7 und 8 einen in der Vorrichtung nach Figuren 2 bis 4 verwendeten Prüßmeßkörper in Aufsicht und Seitenansicht.
Figuren 9 bis 11 eine weitere vorteilhafte Variante eines Prüfmeßkörpers in verschiedenen Ansichten.

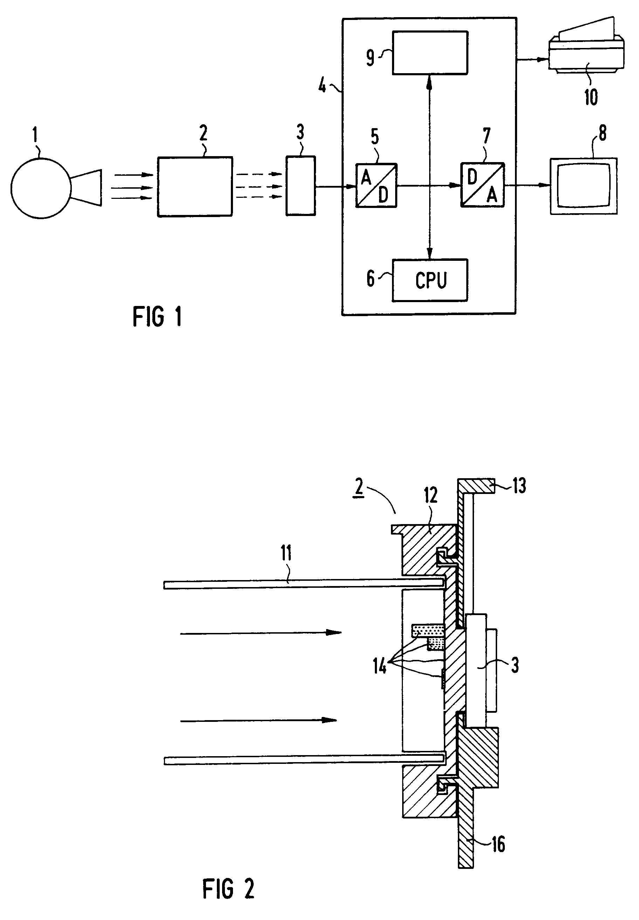

Anhand der Figur 1 wird zunächst das erfindungsgemäße Verfahren näher beschrieben. Die von einer allgemein mit 1 bezeichneten Röntgenstrahlenquelle ausgehende Röntgenstrahlung durchdringt einen anhand der nachfolgenden Figuren noch näher erläuterten Prüfmeßkörper 2 und trifft sodann auf einen Sensor 3, der die Röntgenstrahlung in elektrische Signale umwandelt. Diese werden einem Rechner 4 zugeführt, mittels eines A/D-Wandlers 5 in digitale Signale umgewandelt, die dann in einem Prozessor 6 zu Bildsignalen oder Bildwerten verarbeitet werden. Diese werden anschließend entweder direkt über einen D/A-Wandler 7 einer Anzeigeeinheit 8 in Form eines Monitors zugeführt oder zunächst mit in einem Digitalspeicher 9 abgespeicherten Sollbildwerten verglichen und bei Abweichungen der Anzeigeeinheit 8 zugeführt. Alternativ oder auch additiv zum Monitor kann eine Anzeigeeinheit 10 in Form eines Druckers vorgesehen sein.

Die Figur 2 zeigt in einer Schnittdarstellung eine Ausführungsform des Prüfmeßkörpers 2 zur Abnahme- und Konstanzprüfung eines intraoral einem Patienten applizierbaren Sensors, der von einer extraoral angeordneten Röntgenstrahlenquelle beaufschlagt wird. Bei solchen Dental-Röntgengeräten weist der Röntgenstrahler in der Regel ein zylindrisches Tubusende auf. Auf dieses Tubusende, in Figur 2 mit 11 bezeichnet, ist der Prüfmeßkörper 2 aufgesteckt.

Der Prüfmeßkörper 2 besteht aus zwei Gehäuseteilen 12 und 13, einem auf das Tubusende 11 aufsteckbaren feststehenden Gehäuseteil 12, der mehrere Absorptionselemente 14 aufnimmt, und einem gegenüber dem feststehenden Gehäuseteil 12 drehbaren Gehäuseteil 13, an dem der an sich intraoral einem Patienten applizierbare Sensor 3 in reproduzierbarer Weise gehaltert ist. Die Absorptionselemente 14, die in einer der nachfolgenden Figuren noch näher erläutert werden, sollten so dicht wie möglich vor dem Sensor angeordnet sein, um mögliche Parallaxe-Fehler zu vermeiden.

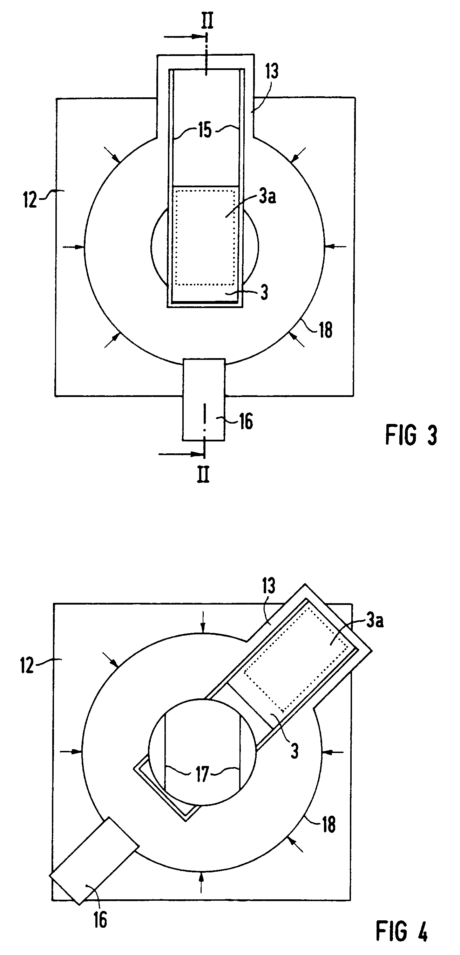

In Verbindung mit der Darstellung nach Figur 3, die den Prüfkörper 2 in Frontansicht zeigt, geht hervor, daß der Sensor 3 bzw. dessen punktiert angedeutete aktive Fläche 3a zentrisch am Tubusausgang angeordnet ist. In der dargestellten Position verläuft der Zentralstrahl also senkrecht durch den Mittelpunkt der aktiven Sensorfläche und parallel zur Flächennormalen. Die winkelige Ausrichtung ist durch eine mit 15 bezeichnete Führung festgelegt.

Das Gehäuseteil 13, in dem der Sensor 3 fixiert ist, kann, wie durch nicht näher bezeichnete Pfeile angedeutet, in 45°-Schritten gedreht und in den jeweiligen Stellungen rastend fixiert werden. Zur Verstellung ist an der Unterseite des Gehäuseteils 13 ein Griff 16 vorgesehen. Ein Verdrehen des Gehäuseteils 13 ist jedoch nur möglich, wenn der Sensor 3 längs der Führung 15, also in radialer Richtung, aus der in Figur 3 gezeigten Grundstellung in die in Figur 4 gezeigte Position gebracht ist. In dieser Position kommt der Sensor 3 außerhalb von in Figur 4 mit 17 bezeichneten, am ortsfesten Gehäuseteil 12 angebrachten seitlichen Führungsteilen zu liegen. In dieser zweiten Position, in der die aktive Fläche 3a des Sensors 3 den mit 18 angedeuteten Strahlenfeldrand überdeckt, kann der Strahlenfeldrand vermessen werden. Der Sensor ist auch hier in reproduzierbarer Weise in der Führung 15 gehaltert. Die aktive Sensorfläche 3a deckt nur zum Teil den Nutzstrahl ab, wodurch die Ausdehnung des Feldrandes analysiert und über den Rechner (beispielsweise über einen PC) vermessen werden kann.

Um den gesamten Rand zu vermessen, ist es erforderlich, den Sensor in mehreren Schritten (hier im Ausführungsbeispiel in 45°-Schritten) über den Rand zu führen. Eine in der Recheneinheit 4 enthaltene Prüf-Software kann alle relevanten Koordinaten für eine Meßanalyse enthalten. Der Abstand Drehmittelpunkt zur aktiven Sensorkante kann von der Prüf-Software abgefragt werden, damit die geometrischen Verhältnisse dem System zur Berechnung bekannt sind. Mit jeder Winkel-Einstellung kann nun eine Aufnahme durchgeführt werden, die vom digitalen System erfaßt, dargestellt und ausgewertet wird. Dabei wird der Abstand zwischen dem Drehmittelpunkt und dem Strahlenfeldrand automatisch vermessen und mit Sollwerten verglichen. Für den eingangs bereits geschilderten Fall einer direkten Anzeige am Monitor 8 kann es vorteilhaft sein, zur Unterstützung der visuellen Überprüfung eine Soll-Feldrand-Zone in Form eines Kreissegments zusätzlich auf dem Monitor in die Prüfaufnahme einzublenden. Aus der Abweichung der ermittelten Radien kann auf die Zentrierung geschlossen werden.

Um eine feinere Auswertung des Strahlenfeldrandes zu bekommen, können die Winkelschritte auch enger gewählt werden.

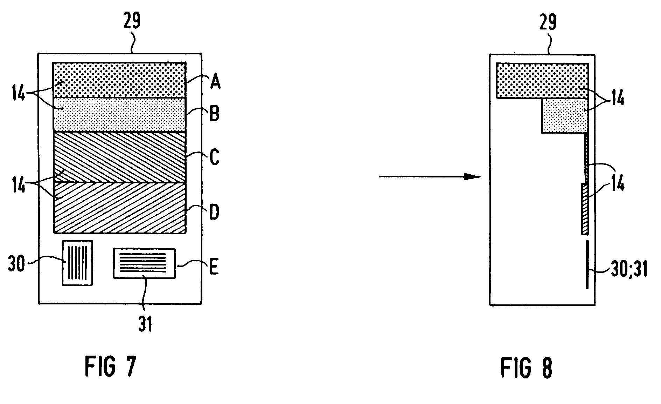

Die Anordnung der Absorptionselemente 14, die der Prüfmeßkörper 2 beinhaltet, ist aus den Figuren 7 und 8 ersichtlich. Sie wird später noch näher beschrieben.

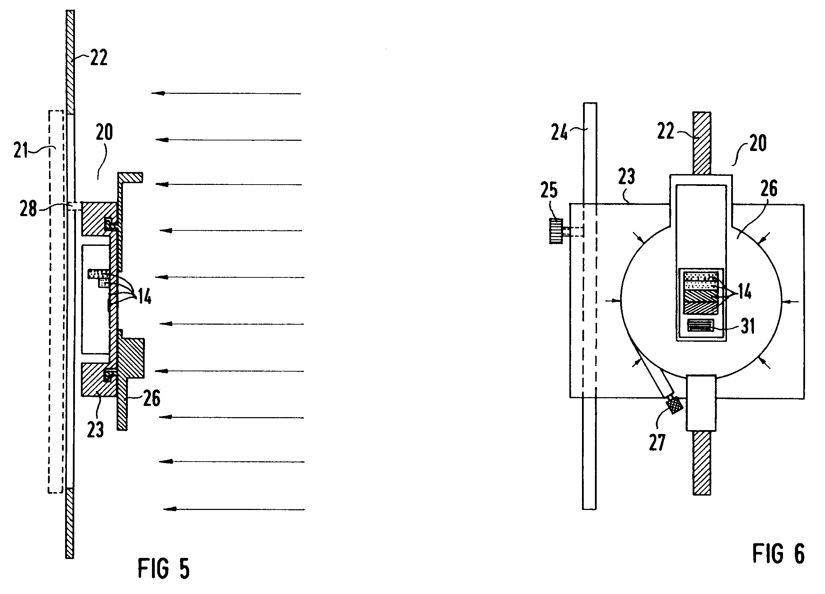

Die Figuren 5 und 6 zeigen eine Ausführungsform, die dazu geeignet ist, eine Abnahme- und Konstanzprüfung bei filmlosen Dental-Röntgengeräten zur Erstellung von Panorama-Schichtaufnahmen in Slot-Technik durchführen zu können. Der hier zur Anwendung gelangende Prüfmeßkörper 20 ist ähnlich aufgebaut wie der zuvor beschriebene Prüfmeßkörper 2; er unterscheidet sich jedoch hinsichtlich seiner Halterung am Röntgengerät sowie hinsichtlich der Anordnung der Bleistrichraster.

Der zu prüfende Sensor 21 ist vorteilhafterweise ein CCD, welches dicht benachbart der Sekundärblende 22 angeordnet ist und deren Schlitz abdeckt. Bei einem solchen CCD werden die Signale durch Integration der erzeugten Ladungen gebildet, wobei die Ladungen entsprechend dem Bewegungsablauf des Panorama-Schichtgerätes quer zum Schlitz der Sekundärblende weitergetaktet werden.

Obgleich der Prüfmeßkörper 20, wie in Figuren 7 und 8 dargestellt, unterhalb der abgestuften Absorptionselemente 14 zwei Bleistrichraster (Pos. 30 und 31) enthalten kann, die um 90° gegeneinander versetzt angeordnet sind (Figur 7), kann es bei der Abnahme und Konstanzprüfung von Panorama-Röntgengeräten vorteilhaft sein, nur ein einziges Bleistrich-Raster mit quer zur Schlitzblende verlaufenden Gittern vorzusehen. Es wird dabei von folgender Überlegung ausgegangen:

Bei Panorama-Schichtaufnahmegeräten verwendet man zur Messung der Kontrastübertragung vorteilhafterweise Bleistrichraster, deren Gitter senkrecht zueinander angeordnet sind. Die Bleistrichraster werden gleichzeitig und überlagerungsfrei im Strahlengang dicht vor der aktiven Fläche des Detektors angeordnet oder nacheinander dorthin gebracht.

Zur Bestimmung der Kontrastübertragung quer zum Sekundärblendenschlitz wird die Röntgenstrahlenintensität durch geeignete Mittel, z.B. durch elektronische Ansteuerung des Generators oder eine rotierende Sektorenblende mit abwechselnd freien und bleibelegten Sektoren, zeitlich moduliert. Die Periodendauer entspricht dabei der Zeit für die Bewegung eines Bildpunktes im Bewegungsablauf des Panorama-Schichtgerätes quer zum Sekundärblendenschlitz um eine Periodenlänge des längs zum Sekundärblendenschlitz angeordneten Gitters. So erhält man modulierte Sensorsignale mit einem der Kontrastübertragung entsprechenden Modulationshub.

Zur Bestimmung der Kontrastübertragung längs zum Sekundärblendenschlitz wird einfach der Sensor durch das quer zum Sekundärblendenschlitz angeordnete Gitter mit Röntgenstrahlung beaufschlagt. Das bei praktischer Anwendung entstehende Problem, daß dabei das Gitter sehr genau in bezug auf die Bewegungsrichtung des Panorama-Schichtaufnahmegerätes justiert sein muß, löst man durch folgende Abwandlungen: Die Messung erfolgt bei gestopptem Bewegungsablauf und genauso gestopptem Sensorsteuerungstakt oder der Sensor wird mit einem einzelnen Röntgenpuls bestrahlt, dessen Pulsdauer relativ kurz ist gegen die Zeit, in der sich ein Bildpunkt quer über den Sekundärblendenschlitz bewegt.

Bei Verwendung eines Bleistrichrasters, dessen Linienabstand nicht groß ist gegen die Ausdehnung einzelner, die Auflösung bestimmender Detektorelemente, hängt die Kontrastübertragung von der (räumlichen) Phasenlase zwischen Rasterlinien und Detektorelementen ab. Hier verdreht man mit Vorteil das Bleistrichraster um einen kleinen Winkelbetrag, z.B. 5°, aus der zu messenden Richtung, so daß über die Ausdehnung des Sensors die verschiedensten Phasenlagen vorkommen, woraus durch rechnergestützte Analyse z.B. die maximale Kontrastübertragung ermittelt werden kann.

Bei der aus Figur 6 ersichtlichen Ausführung ist der Prüfmeßkörper 20 längs des Schlitzes der Sekundärblende 22 verstellbar. Hierzu ist das nicht drehbare Gehäuseteil 23 des Prüfmeßkörpers 20 entlang einer am Röntgengerät befestigten Führungsstange 24 bewegbar und durch eine Feststellschraube 25 in verschiedenen Positionen feststellbar. Die Absorptionselemente 14 und das Bleistrichraster 31 lassen sich so in verschiedene Positionen relativ zum Sensor 21 bringen. Mit Hilfe einer nicht dargestellten Rasteinrichtung, z.B. einer Kugelrasteinrichtung, kann der drehbare Gehäuseteil 26 in 45°-Schritten, also auch um 90°, rastend gedreht werden. Damit sind Messungen parallel und senkrecht zur TDI-Richtung möglich. Um das Bleistrichraster 31 für eine Messung in der oben erläuterten Weise zur Ermittlung der Kontrastübertragung in Position zu bringen, ist außerdem eine Feinverstellung vorgesehen, die es ermöglicht, das Bleistrichraster um etwa 5° zu verstellen, um so verschiedene Phasenlagen zwischen dem Gitter des Bleistrichrasters und der Sensormatrix einstellen zu können. Diese Feineinstellung kann beispielsweise mit Hilfe einer Einstellschraube 27 vorgenommen werden, die auf die vorgenannte Kugelrastvorrichtung einwirkt und diese um die genannten 5 Winkelgrade verschiebt. Alternativ kann auch eine Feineinstellung in Form einer Gewindeverzahnung oder dergleichen vorgesehen sein.

Wie bereits angesprochen, ist es alternativ auch denkbar, zwei Bleistrichraster vorzusehen und den Prüfmeßkörper 20 fest, d.h. nicht längs des Schlitzes der Sekundärblende verstellbar, anzuordnen. Dies kann in geeigneter Weise beispielsweise dadurch geschehen, daß der nicht drehbare Gehäuseteil 23 mit einem Befestigungshaken 28 (in Figur 5 gestrichelt eingezeichnet) versehen ist, der durch den Schlitz der Sekundärblende hindurchgreift und den Prüfkörper in definiertem Abstand zum Sensor fixiert.

Die Absorptionselemente 14 sind in der Weise anzuordnen, daß sie sich im Verlaufe einer Testaufnahme nicht gegenseitig überlagern. Hierzu müssen die Elemente senkrecht zur Integrationsrichtung angeordnet sein.

Die Figuren 7 und 8 lassen in Aufsicht und Seitenansicht die Anordnung der Absorptionselemente des Prüfmeßkörpers bei Anwendung auf Geräte mit intraoral applizierbarem Sensor erkennen. Die Absorptionselemente 14 bilden eine Meßfeldanordnung 29, bei der von oben gesehen zunächst drei Felder A, B und C mit Schwächungskörpern vorgesehen sind, die hinsichtlich ihres Absorptionsverhaltens in Anlehnung an das Röntgenfilmverfahren in der Weise abgestuft sind, daß sie bei einer Filmbelichtung eine Dichteabstufung im Verhältnis 1:1,25:1,5 ÜS (= über Schleier) hervorrufen würden. Diese Abstufung dient dazu, eine Funktion zwischen der Dosis und dem digitalisierten Meßsignal zu ermitteln. Anhand Abweichungen von Abbildungen im Bereich der Stufe A kann auch das Signalrauschen bzw. Abweichungen von einem Standard festgestellt werden. Mit Hilfe der beiden Felder A und D können Abweichungen vom Standard bezüglich des Kontrastes festgestellt werden. Mit Hilfe des Feldes E, welches die bereits erwähnten zwei Bleistrichraster 30, 31 enthält, deren Gitter senkrecht zueinander angeordnet sind, kann die Kontrastübertragung bei einer bestimmten räumlichen Absorptionsmodulation ermittelt werden.

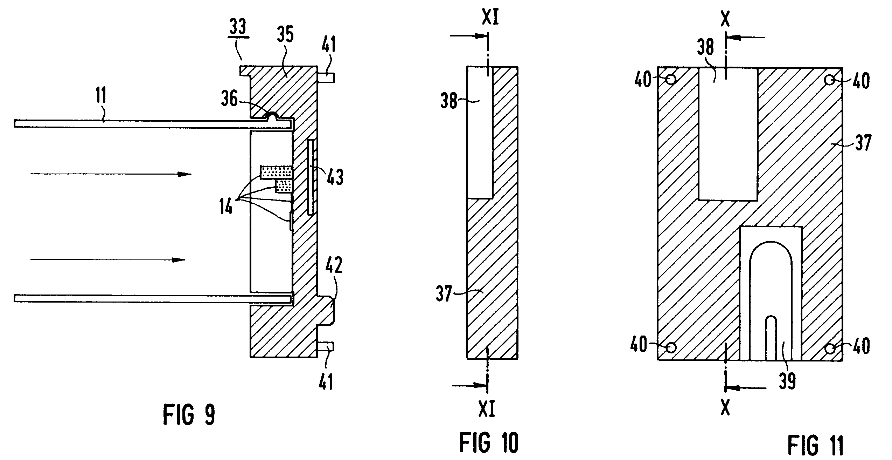

Die Figuren 9 bis 11 zeigen eine weitere vorteilhafte Ausführungsform eines Prüfmeßkörpers. Der Prüfmeßkörper 33 dieser Variante enthält ein Gehäuseteil 35, welches im Gegensatz zu den zuvor erläuterten Varianten nicht ortsfest, sondern in mehreren Schritten, vorzugsweise in 45°-Schritten, gegenüber dem Tubus 11 drehbar und in den einzelnen Positionen rastbar angeordnet ist. Hierzu kann eine geeignete, in Figur 9 mit 36 bezeichnete Rasteinrichtung, z.B. in Form einer Kugelrastung, vorgesehen sein. Auf das Gehäuseteil 35 kann ein in Figur 10 von der Seite und in Figur 11 von vorne im Schnitt dargestelltes zweites Gehäuseteil 37 aufgesetzt werden, welches Träger des verwendeten Sensors ist. Um den Prüfkörper auch für unterschiedlich konturierte Sensoren einsetzen zu können, enthält das Gehäuseteil 37 mehrere unterschiedlich gestaltete Ausnehmungen 38, 39, in die die Sensoren alternativ einsetzbar sind. Die Ausnehmungen 38, 39 sind so angeordnet, daß die aktive Fläche des eingesetzten Sensors einerseits den Absorptionselementen korrespondierend gegenübersteht und andererseits den Strahlenfeldrand überdeckt. Durch mehrere Messungen unter Drehung des Gehäuseteils 35 in der vorgenannten Weise kann der Strahlenfeldrand in seiner Gesamtheit erfaßt und so eine eventuell unzulässige Abweichung von einer festgelegten Norm festgestellt werden.

Das Gehäuseteil 37 enthält zur Fixierung bzw. Halterung am Gehäuseteil 35 vier Bohrungen 40, in die entsprechend gestaltete Zapfen 41 am Gehäuseteil 35 eingreifen. Das Gehäuseteil 37 kann so in zwei um 180° gedrehte Positionen am Teil 35 befestigt werden, wobei ein am Teil 35 vorgesehener Vorsprung 42 den Benutzer daran hindert, den für die Prüfung vorgesehenen Sensor versehentlich in die falsche Ausnehmung (hier die Ausnehmung 39) einzuführen. Wird das Gehäuseteil 37 um 180° gedreht aufgesteckt, greift der Vorsprung 42 in die Ausnehmung 38 und verhindert so das Einführen des Sensors in diese Ausnehmung. Ein weiteres Unterscheidungsmerkmal dieser Ausführungsform ist, daß das Gehäuseteil 35 noch mit einem Querschlitz 43 versehen ist, der so ausgebildet und angeordnet ist, daß ein üblicherweise verwendeter Röntgenfilm, typisch sind die Formate 2 x 3 cm bis 3 x 4 cm, einführbar und in bezug auf die Absorptionselemente positionierbar ist. Damit ist eine Konstanzprüfung nicht nur für filmlose Dental-Röntgengeräte, sondern auch für Geräte mit Röntgenfilm, wie sie heute in Gebrauch sind, durchführbar.

## Patentansprüche

1. Verfahren zur Abnahme- und Konstanzprüfung filmloser Dental-Röntgengeräte bei dem in den Strahlengang der Röntgenstrahlen ein Prüfmeßkörper (2, 20, 33) mit mehreren Absorptionselementen (14) unterschiedlicher Absorption in einem definierten engen Abstand zu einem strahlenempfindlichen Sensor (3, 21) angeordnet wird, bei dem die aus dem Sensor gewonnenen elektrischen Signale einer Recheneinheit (4) zugeführt werden, welche diese zu Bildwertsignalen verarbeitet, die dann entweder direkt einer Anzeigeeinheit (8, 10 ) zugeführt werden oder zunächst mit vorgegebenen Sollbildwerten verglichen und bei Abweichungen der Anzeigeeinheit (8, 10 ) zugeführt werden.

2. Vorrichtung zur Durchführung des Verfahrens nach Anspruch 1 , bei der zur Abnahme- und Konstanzprüfung eines Dental-Röntgengerätes zur Erstellung von intraoralen Röntgenaufnahmen unter Benutzung eines strahlenempfindlichen Sensors (3, 21), der intraoral eines Patienten applizierbar ist, am Tubus des Röntgengerätes ein Prüfmeßkörper (2, 20, 33) positionierbar ist, welcher mit mehreren, in einem definierten engen Abstand zum Sensor (3, 21) angeordenten Absorptionselementen (14) unterschiedlicher Absorption versehen ist und welcher Mittel (13, 15, 38, 39) zur reproduzierbaren Halterung des Sensors (3, 21) enthält.

3. Vorrichtung zur Durchführung des Verfahrens nach Anspruch 1 , bei der zur Abnahme- und Konstanzprüfung eines Dental-Röntgengerätes zur Erstellung von Panorama-Röntgenaufnahmen, unter Benutzung eines strahlenempfindlichen Sensors (3, 21), der extraoral eines Patienten applizierbar ist, an der Sekundärblende (22) des Panorama-Röntgengerätes ein Prüfmeßkörper (2, 20, 33) vorgesehen ist, welcher mit mehreren, in einem definierten engen Abstand zum Sensor (3,21) angeordneten Absorptionselementen (14) unterschiedlicher Absorption versehen ist und welcher Mittel (24 bis 26; 28) zur reproduzierbaren Halterung des Sensors ( 3, 21) enthält.

4. Vorrichtung nach Anspruch 2, bei der die Halterungsmittel (13, 15, 38, 39) so ausgebildet sind, daß der Sensor (3) aus einer zentrisch zu der durch die Absorptionselemente (14) gebildeten Meßfeldanordnung (29) angeordneten ersten Position in eine dezentral dazu angeordnete zweite Position bringbar ist, in der die aktive Fläche (3a) des Sensors (3) den Strahlenfeldrand (18) der Strahlenaustrittsfläche erfaßt.

5. Vorrichtung nach Anspruch 4, bei der die Haltemittel für den Sensor (3) eine radial sich erstreckende Führung (15) beinhalten.

6. Vorrichtung nach Anspruch 4 oder 5, bei der die Absorptionselemente (14) in einem ersten, am Tubusende (11) des Röntgengerätes zu halternden Gehäuseteil (12, 35) und die Haltemittel (15) für den Sensor (3) in einem gegenüber dem ersten Gehäuseteil drehbaren zweiten Gehäuseteil (13, 37) angeordnet sind.

7. Vorrichtung nach Anspruch 6, bei der die Drehbarkeit 360° beträgt und der Winkelbereich in mehrere rastbare Abschnitte (Sektoren) unterteilt ist.

8. Vorrichtung nach einem der Ansprüche 2 bis 7, bei der die Haltemittel ein Gehäuseteil (37) umfassen, welches mehrere Ausnehmungen (38, 39) zur alternativen Aufnahme mehrerer, unterschiedlich konturierter Sensoren (3) enthält, wobei das Gehäuseteil (37) durch Positioniermittel (40, 41) in verschiedenen Positionen in bezug auf das auf den Tubus aufsetzbare Gehäuseteil (35) und korrespondierend zu den Absorptionselementen (14) bringbar ist, wobei Sperrmittel (42) vorgesehen sind, die das Einführen eines Sensors in eine den Absorptionselementen (14) nicht korrespondierend gegenüberstehende Ausnehmung (39) verhindern.

9. Vorrichtung nach Anspruch 8, bei der das auf den Tubus aufsetzbare Gehäuseteil (35) gegenüber dem Tubus drehbar angeordnet ist.

10. Vorrichtung nach Anspruch 9, bei der die Drehstellungen durch Markierungen oder Raststellungen (36) reproduzierbar einzustellen sind.

11. Vorrichtung nach einem der Ansprüche 8 bis 10, bei der das auf den Tubus (11) aufsetzbare Gehäuseteil (35) einen Schlitz (43) zum Einführen eines konventionellen Intraoral-Röntgenfilmes aufweist.

## Claims

1. Method for acceptance testing and constancy testing of filmless dental X-ray apparatuses, in which method a test measurement body (2, 20, 33) having a plurality of absorption elements (14) which have different levels of absorption is arranged in the beam path of the X-rays at a defined narrow distance from a radiation-sensitive sensor (3, 21), in which method the electrical signals obtained from the sensor are supplied to a central processor (4), which processes these signals to form image value signals, which are then either supplied directly to a display unit (8, 10) or are first of all compared with pre-determined desired image values and are supplied to the display unit (8, 10) in the event of deviations.

2. Device for carrying out the method according to claim 1, in which device, for acceptance testing and constancy testing of a dental X-ray apparatus for preparing intra-oral x-ray pictures using a radiation-sensitive sensor (3, 21) which can be applied inside the mouth of a patient, a test measurement body (2, 20, 33) can he positioned on the tube of the X-ray apparatus, which test measurement body is provided with a plurality of absorption elements (14), which have different levels of absorption and are arranged at a defined narrow distance from the sensor (3, 21), and which test measurement body contains means (13, 15, 38, 39) forming a reproducible holding device for the sensor (3, 21).

3. Device for carrying out the method according to claim 1, in which device, for acceptance testing and constancy testing of a dental X-ray apparatus for preparing panoramic X-ray pictures using a radiation-sensitive sensor (3, 21) which can be applied outside the mouth of a patient, a test measurement body (2, 20, 33) is provided on the secondary diaphragm (22) of the panoramic X-ray apparatus, which test measurement body is provided with a plurality of absorption elements (14), which have different levels of absorption and are arranged at a defined narrow distance from the sensor (3, 21), and which test measurement body contains means (24 to 26; 28) forming a reproducible holding device for the sensor (3, 21).

4. Device according to claim 2, in which the holding means (13, 15, 38, 39) are constructed in such a way that the sensor (3) can be brought out of a first position, which is arranged centrally with respect to the measuring field arrangement (29) formed by the absorption elements (14), into a second position which is arranged decentrally with respect thereto and in which the active surface (3a) of the sensor (3) detects the edge (18) of the radiation field of the radiation-emission surface.

5. Device according to claim 4, in which the holding means for the sensor (3) include a radially-extending guide (15).

6. Device according to claim 4 or 5, in which the absorption elements (14) are arranged in a first housing portion (12, 35) which is to be held at the tube end (11) of the X-ray apparatus, and the holding means (15) for the sensor (3) are arranged in a second housing portion (13, 37) which is rotatable with respect to the first housing portion.

7. Device according to claim 6, in which the turnability amounts to 360° and the angular range is divided into a plurality of lockable sections (sectors).

8. Device according to one of the claims 2 to 7, in which the holding means comprise a housing portion (37), which contains a plurality of recesses (38, 39) for alternative acceptance of a plurality of sensors (3) having different contours, with the housing portion (37) being able to be brought by positioning means (40, 41) into various positions in relation to the housing portion (35) which can be mounted on the tube, and consequently towards the absorption elements (14), with blocking means (42) being provided, which blocking means prevent the insertion of a sensor into a recess (39) which is not correspondingly opposite the absorption elements (14).

9. Device according to claim 8, in which the housing portion (35) which can be mounted on the tube is arranged so as to be rotatable with respect to the tube.

10. Device according to claim 9, in which the rotary positions are to be reproducibly adjusted by means of markings or locking positions (36).

11. Device according to one of the claims 8 to 10, in which the housing portion (35) which can be mounted on the tube (11) has a slit (43) for inserting a conventional intra-oral X-ray film.

## Revendications

1. Procédé pour effectuer des essais de réception et un test de constance d'appareils radiographiques dentaires sans film, dans lequel il est monté dans le trajet des rayons X un échantillon (2, 20, 33) de mesure comportant plusieurs éléments (14) absorbants d'absorption différente à une petite distance définie d'un capteur (3, 21) sensible au rayonnement, dans lequel les signaux électriques obtenus par le capteur sont envoyés à une unité (4) de calcul qui les traite pour en faire des signaux d'éléments d'image qui sont ensuite envoyés directement à une unité (8 10) d'affichage ou d'abord comparés à des éléments d'image de consigne et, en cas d'écarts, envoyés à l'unité (8, 10) d'affichage.

2. Dispositif de mise en oeuvre du procédé suivant la revendication 1, dans lequel, pour effectuer des essais de réception et un test de constance d'un appareil radiographique dentaire servant à produire des prises de vues radiographiques intraorales, tout en utilisant un capteur sensible (3, 21) au rayonnement, qui peut être appliqué à un patient de manière intraorale, il peut être mis en position sur le tube de l'appareil radiographique un échantillon (2, 20, 33) de mesure qui est muni de plusieurs éléments (14) absorbants d'absorption différente, montés à une petite distance définie du capteur (3, 21), et qui comporte des moyens (13, 15, 38, 39) pour fixer le capteur (3, 21) de manière reproductible.

3. Dispositif de mise en oeuvre du procédé suivant la revendication 1, dans lequel, pour effectuer des essais de réception et un test de constance d'un appareil radiographique dentaire servant à produire des prises de vues de tomographie, tout en utilisant un capteur sensible (3, 21) au rayonnement, qui peut être appliqué à un patient de manière extraorale, il est prévu sur le diaphragme (22) secondaire de l'appareil de tomographie un échantillon (2, 20, 33) de mesure qui est muni de plusieurs éléments (14) absorbants d'absorption différente, montés à une petite distance définie du capteur (3, 21), et qui comporte des moyens (24 à 26; 28) pour fixer le capteur (3, 21) de manière reproductible.

4. Dispositif suivant la revendication 2, dans lequel les moyens (13, 15, 38, 39) de fixation sont réalisés de manière que le capteur (3) puisse être amené d'une première position qui est centrée par rapport à la disposition (29) de zones de mesure formée par les éléments (14) absorbants à une deuxième position qui est décentrée par rapport à ladite disposition et en laquelle la surface (3a) active du capteur (3) détecte le bord du champ de rayonnement de la surface de sortie des rayons.

5. Dispositif suivant la revendication 4, dans lequel les moyens de maintien du capteur (3) comportent une glissière (15) s'étendant radialement.

6. Dispositif suivant la revendication 4 ou 5, dans lequel les éléments (14) absorbants sont disposés dans une première pièce (12, 35) de boîtier à fixer à l'extrémité (11) du tube de l'appareil radiographique et dans lequel les éléments (15) de maintien du capteur (3) sont disposés dans une deuxième pièce (13, 37) de boîtier que l'on peut faire tourner par rapport à la première pièce de boîtier.

7. Dispositif suivant la revendication 6, dans lequel la possibilité de rotation est de 360° et l'intervalle angulaire est subdivisé en plusieurs parties (secteurs) encliquetables.

8. Dispositif suivant l'une des revendications 2 à 7, dans lequel les moyens de maintien comportent une pièce (37) de boîtier qui comprend plusieurs évidements (38, 39) servant à recevoir alternativement plusieurs capteurs (3) de contour différent, la pièce (37) de boîtier pouvant être mise par des moyens (40, 41) de mise en position en diverses positions par rapport à la pièce (35) de boîtier enfilée sur le tube et de manière correspondante par rapport aux éléments (14) absorbants, des moyens (42) de blocage étant prévus, qui empêchent l'introduction d'un capteur dans un évidement faisant face aux éléments (14) absorbants d'une manière qui ne correspond pas.

9. Dispositif suivant la revendication 8, dans lequel la pièce (35) de boîtier enfilée sur le tube est montée tournante par rapport au tube.

10. Dispositif suivant la revendication 9, dans lequel les positions angulaires sont à régler de manière reproductible par des repères ou des emplacements (36) d'encliquetage.

11. Dispositif suivant l'une des revendications 8 à 10, dans lequel la pièce (35) de boîtier enfilée sur le tube comporte une fente (43) pour introduire un roentgen-film intraoral traditionnel.
